(19) Europäisches Patentamt
European Patent Office
Office européen des brevets

(11) Veröffentlichungsnummer: **0 358 116**
**A2**

# (12) EUROPÄISCHE PATENTANMELDUNG

(21) Anmeldenummer: **89116131.7**

(22) Anmeldetag: **31.08.89**

(51) Int. Cl.5: **C07D 513/14 , A61K 31/415 , //(C07D513/14,333:00,277:00, 235:00)**

Patentansprüche für folgende Vertragsstaaten: ES + GR.

(30) Priorität: **08.09.88 AT 2202/88**

(43) Veröffentlichungstag der Anmeldung: **14.03.90 Patentblatt 90/11**

(84) Benannte Vertragsstaaten: **AT BE CH DE ES FR GB GR IT LI LU NL SE**

(71) Anmelder: **CHEMISCH PHARMAZEUTISCHE FORSCHUNGS-GESELLSCHAFT m.b.H.**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Rovenszky, Franz, Dipl.-Ing.-Dr.**
**Lagerhausstrasse 5/8**
**A-2460 Bruck a.d.Leitha(AT)**
Erfinder: **Ferber, Hubert Peter, Dr.**
**Ahornweg 24**
**A-4021 Ansfelden(AT)**

(74) Vertreter: **Kunz, Ekkehard, Dr.**
**Chemie Holding AG Patentwesen St. Peter-Strasse 25**
**A-4021 Linz(AT)**

(54) **Neue Thieno(3',4'-4,5)imidazo(2,1-b)-thiazol-Derivate, ein Verfahren zu ihrer Herstellung und ihre Verwendung in Medikamenten.**

(57) Die Erfindung betrifft neue Thieno-(3',4'-4,5)imidazo(2,1-b)thiazol-Derivate der Formel

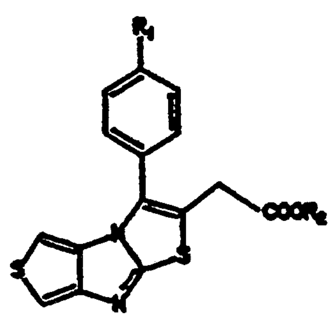

I,

in der
$R_1$ Wasserstoff, Halogen oder $CF_3$ und $R_2$ Wasserstoff oder $C_1$ - $C_4$ Alkyl bedeuten und, für den Fall, daß $R_2$ Wasserstoff bedeutet, ihre pharmazeutisch verwendbaren Salze, ein Verfahren zur Herstellung dieser Verbindungen und deren Anwendung zur Behandlung von Krebs oder rheumatischer Arthritis, hervorgerufen durch ein defektes Immunsystem.

EP 0 358 116 A2

# Neue Thieno(3',4'-4,5)imidazo(2,1-b)thiazol-Derivate, ein Verfahren zu ihrer Herstellung und ihre Verwendung in Medikamenten

Die Erfindung betrifft neue Thieno(3',4'-4,5)imidazo(2,1-b)thiazol-Derivate, ein Verfahren zu ihrer Herstellung und ihre Verwendung in Medikamenten zur Stimulierung des Immunsystems.

S. C. Gilman et al. beschreiben in Agents and Actions, Vol. 17, 1 (1985) 3-(p-Chlorphenyl)thiazol(3,2-a)-benzimidazol-2-essigsäure als entzündungshemmende und immunmodulierende Substanz.

Es wurde nun gefunden, daß Thieno(3',4'-4,5)imidazo(2,1-b)thiazol-Derivate eine gegenüber dem Stand der Technik verbesserte pharmakologische Wirkung besitzen.

Gegenstand der Erfindung sind daher neue Verbindungen der Formel

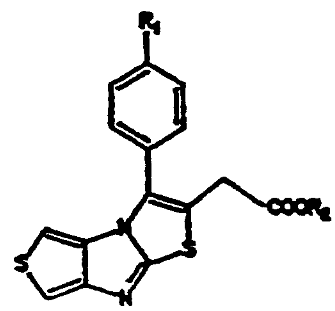

I,

in der
$R_1$ Wasserstoff, Halogen oder Trifluormethyl und $R_2$ Wasserstoff oder $C_1$ - $C_4$ Alkyl bedeuten und, für den Fall, daß $R_2$ Wasserstoff bedeutet, ihre pharmazeutisch verwendbaren Salze.

Der in dieser Beschreibung verwendete Ausdruck "$C_1$ - $C_4$ Alkyl" bezeichnet geradkettige oder verzweigte gesättigte Kohlenwasserstoffreste mit 1 bis 4 Kohlenstoffatomen, wie z.B. Methyl, Ethyl, Propyl, Isopropyl, Butyl, Isobutyl, tert.Butyl. Der Ausdruck "Halogen" bezeichnet Chlor, Brom oder Fluor.

Eine bevorzugte Klasse der Verbindungen der Formel I ist jene, in der $R_1$ Chlor und $R_2$ Wasserstoff oder Methyl bedeutet.

Besonders bevorzugte Einzelverbindungen sind:
3-(4-Chlorphenyl)-thieno(3',4'-4,5)imidazo(2,1-b)thiazol-2-essigsäuremethylester
3-(4-Chlorphenyl)-thieno(3',4'-4,5)imidazo(2,1-b)thiazol-2-essigsäure

Die Thieno(3',4'-4,5)imidazo(2,1-b)thiazol-Derivate der Formel I und deren Salze werden erfindungsgemäß dadurch hergestellt, daß man

a) eine Verbindung der Formel

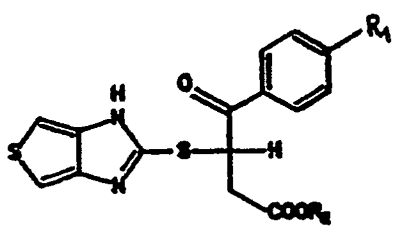

II,

in der $R_1$ die obige Bedeutung hat und $R_2$ $C_1$ - $C_4$ Alkyl bedeutet, oder ein Säureadditionssalz davon in Gegenwart von wasserentziehenden Reagenzien cyclisiert, worauf man

b) gegebenenfalls eine so erhaltene Verbindung der Formel I, in der $R_2$ $C_1$ -$C_4$ Alkyl bedeutet, zu einer Verbindung der Formel I, in der $R_2$ Wasserstoff bedeutet, alkalisch verseift und

c) erwünschtenfalls eine im Verfahrensschritt b) erhaltene freie Säure der Formel I, in der $R_2$ Wasserstoff bedeutet, mit anorganischen oder organischen Basen in ein pharmazeutisch verträgliches Salz überführt.

Als wasserentziehende Reagenzien bei der Cyclisierung von Verbindungen der Formel II können alle

üblicherweise verwendeten wasserentziehenden Mittel verwendet werden. Vorzugsweise kommen Polyphosphorsäure oder Phosphoroxychlorid in Frage, die zugleich als Lösungsmittel verwendet können. Besonders bevorzugt ist Phosphoroxychlorid. Die Cyclisierungstemperatur soll ca. 60 °C bis 110 °C betragen. Bei Phosphoroxychlorid cyclisiert man am besten bei Rückflußtemperatur. Die Reaktionszeit beträgt, abhängig von der Temperatur und dem Cyclisierungsmittel, etwa zwischen 5 Minuten und 4 Stunden, bevorzugt 5 bis 30 Minuten.
Die Aufarbeitung des Reaktionsgemisches erfolgt nach üblichen Reinigungsmethoden wie Umkristallisation, Extraktion, Säulenchromatographie u.ä.. Bevorzugt wird die Extraktion mit Ethylacetat nach destillativer Entfernung des überschüssigen Phosphoroxychlorides und Neutralisation der erhaltenen Lösung durchgeführt.

Gegebenenfalls können die Ester der Formel I durch Kochen mit Basen, vorzugsweise mit äquivalenten Mengen Alkalihydroxidlösungen für 5 Minuten bis 1 Stunde und vorteilhafterweise unter Zusatz eines Lösungsvermittlers wie z.B. Methanol oder Ethanol, in fast quantitativer Ausbeute zu Verbindungen der Formel I, in der $R_2$ Wasserstoff bedeutet, verseift werden. Besonders bevorzugt erfolgt die Verseifung dadurch, daß man den Ester in Methanol suspendiert, mit einer äquivalenten Menge an verdünnter Natronlauge versetzt und für ca. 5 bis 25 Minuten unter Rückfluß erhitzt.

Die bei der Umsetzung nach Verfahrensschritt b) erhaltenen Verbindungen der Formel I, welche eine freie Carboxylgruppe haben, können auf übliche Weise mit anorganischen oder organischen Basen in ihre pharmazeutisch verwendbaren Salze übergeführt werden. Die Salzbildung kann beispielsweise durchgeführt werden, indem man die genannten Verbindungen der Formel I, in der $R_2$ Wasserstoff bedeutet, in einem geeigneten Lösungsmittel, wie beispielsweise Wasser oder einem niederen aliphatischen Alkohol, löst, eine äquivalente Menge der gewünschten Base zusetzt, für eine gute Durchmischung sorgt und nach beendeter Salzbildung das Lösungsmittel im Vakuum abdestilliert. Gegebenenfalls können die Salze der Isolierung umkristallisiert werden.

Pharmazeutisch verwendbare Salze können Metallsalze sein, insbesondere Alkalimetall- oder Erdalkalimetallsalze, wie Natrium-, Kalium-, Magnesium- oder Calciumsalze. Andere pharmazeutisch verwendbare Salze sind beispielsweise auch leicht kristallisierende Ammoniumsalze. Letztere werden abgeleitet von Ammoniak oder organischen Aminen, z.B. Mono-, Di- oder Tri-nieder-(alkyl, cycloalkyl oder -hydroxyalkyl)-aminen, Niederalkylendiaminen oder (Hydroxyniederalkyl oder Arylniederalkyl)-niederalkylammonium Basen, z.B. Methylamin, Diethylamin, Triethylamin, Dicyclohexylamin, Triethanolamin, Ethylendiamin, Tris-(hydroxymethyl)-amino-methan, Benzyl-trimethylammoniumhydroxid und dergleichen.

Die Verbindungen der allgemeinen Formel II können, ausgehend von den literaturbekannten Verbindungen der Formeln III(F. Outerquin und C. Paulmier, Bull. Soc. Chim. Fr., 5-6, 159-163 (1983)) und V (C.F.H. Allen, J.B. Normington und C.V. Wilson, Can.J. Research 11, 382 (1934)), gemäß dem folgenden Reaktionsschema nach üblichen und jedem Fachmann geläufigen chemischen Arbeitsmethoden synthetisiert werden:

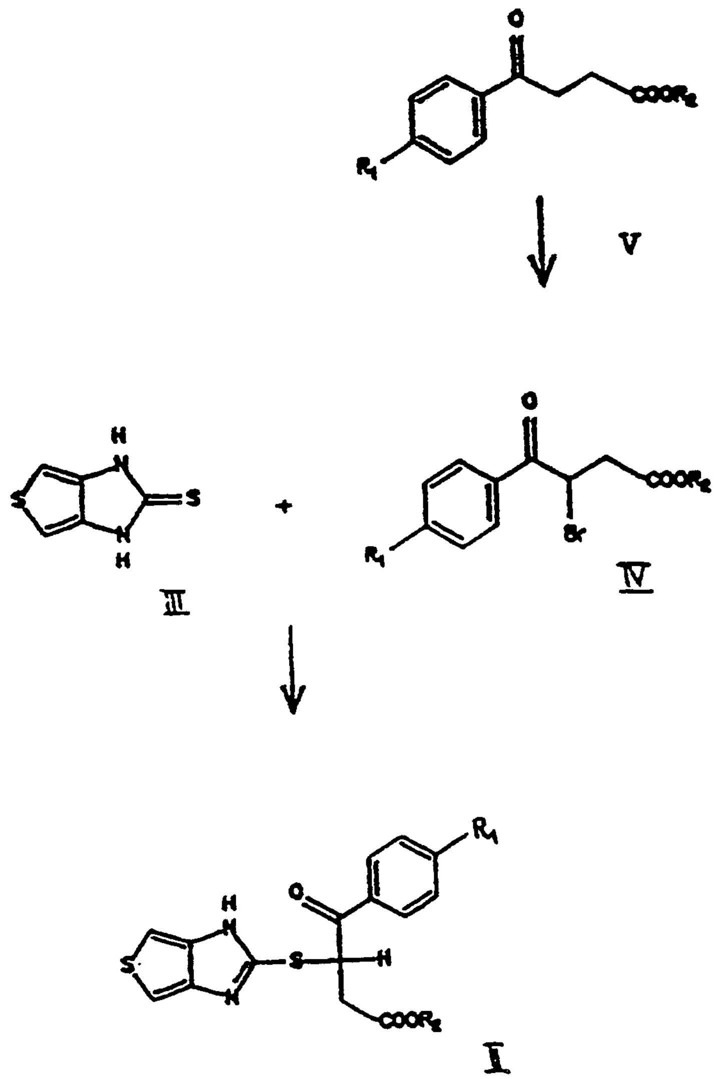

V

III

IV

I

Die neuen Verbindungen der Formel I und ihre pharmazeutisch verwendbaren Salze zeigen in in vitro-Modellen eine hervorragende Stimulierung des Immunsystems. Diese Stimulierung des Immunsystems kann beispielsweise durch Messung der entzündungshemmenden Aktivität der Testsubstanzen im Adjuvansinduzierten Ratten-Polyarthritis-Test gemessen werden.

In diesem Testsystem wurde die entzündungshemmende Wirkung der Substanz des Beispiels 2 der vorliegenden Anmeldung (Verbindung A) mit 3-(4-Chlorphenyl)-thiazolo(3,2-a)benzimidazol-2-essigsäure (Tilomisol) verglichen (Beispiel 3). Aus diesem Vergleich sieht man, daß die Verbindung der vorliegenden Erfindung Tilomisol klar überlegen ist.

Aufgrund dieser pharmakologischen Eigenschaften können die neuen Verbindungen allein oder in Mischung mit anderen Wirksubstanzen in Form üblicher galenischer Zubereitungen bei Erkrankungen, die durch ein defektes Immunsystem verursacht werden, wie z.B. Krebs oder rheumatoider Arthritis, als Medikament verwendet werden.

Die Verbindungen der Formel I sind zur Verwendung am Menschen bestimmt und können auf übliche Weise, wie beispielsweise oral oder parenteral, verabreicht werden. Vorzugsweise werden sie oral verabreicht, wobei die Tagesdosis ca. 0,1 bis 100 mg/kg Körpergewicht beträgt, vorzugsweise 0,2 bis 20 mg/kg Körpergewicht. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben.

Falls die erfindungsgemäßen Substanzen zur Prophylaxe verwendet werden, bewegen sich die dosen ungefähr im selben Rahmen wie im Behandlungsfall. Die orale Verabreichung ist auch im Fall der Prophylaxe bevorzugt.

Die Verbindungen der Formel I können allein oder in Verbindung mit anderen pharmazeutisch aktiven Substanzen verabreicht werden, wobei der Gehalt der Verbindungen der Formel I zwischen 0,1 und 99 %

liegt. Im allgemeinen liegen die pharmazeutisch aktiven Verbindungen in einer Mischung mit geeigneten inerten Hilfs- und/oder Trägerstoffen oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.
Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs- oder Emulgiermittel, Salze zur Veränderung des osmotischen Druckes und dergleichen.

Insbesondere können pharmazeutische Präparate die erfindungsgemäßen Verbindungen in Kombination mit andern therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

Beispiel 1:

3-(4-Chlorphenyl)-thieno(3′,4′-4,5)imidazo(2,1-b)thiazol-2-essigsäuremethylester

8,00 g (17,3 mmol) 4-(4-Chlorphenyl)-4-oxo-3-(1H-thieno(3,4-d)-imidazol-2-yl)-thio-butansäuremethylester, Hydrobromid werden in 75 ml Phosphoroxytrichlorid suspendiert und 10 Minuten zum Sieden erhitzt. Das überschüssige Phosphoroxytrichlorid wird abdestilliert und der Rückstand mit gesättigter Natriumhydrogencarbonatlösung neutralisiert. Danach wird dreimal mit insgesamt 500 ml Ethylacetat extrahiert. Die vereinten organischen Phasen werden über Natriumsulfat getrocknet, filtriert und eingedampft. Der Rückstand wird aus Aceton umkristallisiert.
Ausbeute: 2,00 g (31,8 % d.Th.)
Fp: 188 - 190 °C (Aceton)
$^1$H-NMR: (DMSO)
delta (ppm): 7,68 (s, 4H, Ph-H); 7,22; 7,19; 6,54; 6,51 (AB, 2H, Th-H); 3,81 (s, 2H, $-CH_2-COO-$), 3,67 (s, 3H, $-COOCH_3$)

Das Ausgangsmaterial kann wie folgt hergestellt werden:

3-Brom-4-(4-chlorphenyl)-4-oxo-butansäuremethylester

50,0 g (0,221 mol) 4-(4-chlorphenyl)-4-oxo-butansäuremethylester (C.F.H. Allen, J.B. Normington und C.V. Wilson, Can. J. Research 11, 382 (1934)) werden in 250 ml Eisessig gelöst und drei Tropfen einer Lösung von Bromwasserstoff in Eisessig zugesetzt. Unter Rühren werden 35,3 g (0,221 mol) Brom so zugetropft, daß sich im Reaktionsgemisch keine merkbare Braunfärbung ausbilden kann. Nach beendeter Zugabe wird noch 15 Minuten weitergerührt. Danach wird der Eisessig weitgehend abdestilliert.
Der Rückstand wird mit gesättigter Natriumhydrogencarbonatlösung neutralisiert und dreimal mit insgesamt 600 ml Methylenchlorid extrahiert. Die vereinten organischen Phasen werden über Natriumsulfat getrocknet, filtriert und eingedampft (67,2 g gelbes Öl). Es wird mit Methanol angerieben und aus Methanol umkristallisiert.
Ausbeute: 65,2 g farblose Kristalle (97 % d.Th.)
Fp: 48 - 49 °C (Methanol)

4-(4-Chlorphenyl)-4-oxo-3-(1H-thieno(3,4-d)imidazol-2-yl)thio-butansäuremethylester, Hydrobromid

4,00 g (25,6 mmol) 1,3-Dihydro-thieno(3,4-d)imidazol-2-thion und 7,11 g (23,3 mmol) 3-Brom-4-(4-chlorphenyl)-4-oxo-butansäure-methylester werden in 100 ml absolutem Methanol gelöst und 1,5 Stunden unter Rückfluß erhitzt. Danach wird die Lösung mit ca. 1 g Aktivkohle versetzt, filtriert und eingedampft. Der erhaltene Rückstand wird mit Diethylether digeriert.
Ausbeute: 9,10 g beige Kristalle (84,6 % d.Th.)
Fp: 180 - 185 °C Zers. (Aceton)

Beispiel 2:

3-(4-Chlorphenyl)-thieno(3',4'-4,5)imidazo(2,1-b)thiazol-2-essigsäure

2,00 g (5,51 mmol) 3-(4-Chlorphenyl)-thieno(3',4'-4,5)imidazo(2,1-b)thiazol-2-essigsäuremethylester werden in 20 ml Methanol suspendiert, mit 3 ml 2n wäßriger Natronlauge versetzt und 15 Minuten unter Rückfluß erhitzt. Das Reaktionsgemisch wird auf ca. 10 ml eingeengt und mit 2n Salzsäure angesäuert. Das ausgefallene Produkt wird abgesaugt, dreimal mit destilliertem Wasser gewaschen und aus Methanol umkristallisiert.
Ausbeute: 1,50 g farblose Kristalle (78 % d.Th.)
Fp: 200 - 210 °C Zers. (Methanol)
$^1$H-NMR: (DMSO) delta (ppm): 8,87 (s, breit, 1H, -COOH) 7,72 (s, 4H, Ph-H); 7,42; 7,39; 6,77; 6,74 (AB, 2H, Th-H); 3,83 (s, 2H, $-CH_2-COO-$).

Beispiel 3

Untersuchung der immunmodulierenden Wirkung im Adjuvans- induzierten Ratten - Polyarthritis - Test.

Die pharmakologische Wirkung von 3-(4-Chlorphenyl)-thieno(3',4'-4,5)imidazo(2,1-b)thiazol-2-essigsäure (Verbindung A, = die Verbindung des Beispiels 2 der vorliegenden Anmeldung) wurde im Vergleich mit 3-(4-Chlorphenyl)thiazolo(3,2-a)benzimidazol-2-essigsäure (Tilomisol, Verbindung B) im Adjuvans -induzierten Ratten - Polyarthritis - Test gemessen.

Bei diesem Test wurden weiblichen Lewis-Ratten, die einen angeborenen Immundefekt haben, die Versuchssubstanzen täglich über einen Zeitraum von 16 Tagen intraperitoneal in einer Konzentration von 10 mg/kg Körpergewicht verabreicht. Pro Substanz wurden 6 Tiere eingesetzt. Als Kontrolle dienten Tiere, die 0,5 % Carboxymethylcellulose anstelle der Wirksubstanz erhielten. Am Tag Null (d.h. einen Tag vor Beginn der Verabreichung der Versuchssubstanzen) wurden je Versuchstier 0,75 mg Mycobacterium butyricum in 0,1 ml Freundschem Adjuvans subplantar in die rechte Pfote injiziert. Ab dem 11. - 14. Tag kam es in der linken Pfote, in die nichts injiziert wurde, zu einer Sekundärreaktion, die sich in einer Schwellung der Pfote äußerte. Da diese Schwellung durch eine Immunreaktion hervorgerufen wird, werden Substanzen, die diese genetisch bedingte und krankhafte Immunreaktion reduzieren, als Immunmodulatoren bezeichnet. Die Größe der Schwellung wurde täglich plethysmometrisch gemessen und in ml angegeben.

Die Ergebnisse dieser Versuche sind in Tabelle 1 und Abb. 1 zusammengefaßt.

Tabelle 1

| Tag | Pfotenvolumen im ml | | |
|---|---|---|---|
| | Kontrolle (0,5 % CMC) | Tilomisol 10 mg/kg i.p. | Verbindung A 10 mg/kg i.p. |
| 0 | 0,39 ± 0,02 | 0,39 ± 0,02 | 0,39 ± 0,02 |
| 11 | 0,41 ± 0,02 | 0,45 ± 0,02 °°° | 0,38 ± 0,02 ** |
| 12 | 0,41 ± 0,03 | 0,46 ± 0,02 °° | 0,39 ± 0,02 |
| 13 | 0,41 ± 0,02 | 0,45 ± 0,02 °° | 0,39 ± 0,01 * |
| 14 | 0,47 ± 0,07 | 0,45 ± 0,01 | 0,44 ± 0,02 |
| 16 | 0,50 ± 0,05 | 0,53 ± 0,05 | 0,46 ± 0,03 * |

° signifikanter Anstieg * signifikanter Abfall
CMC: Carboxymethylcellulose

Wie aus Tabelle 1 und Abbildung 1 ersichtlich ist, hemmt nur Verbindung A die auf die Immunreaktion zurückgehende Sekundärreaktion zu allen untersuchten Zeitpunkten.

**Ansprüche**

1. Neue Thieno-(3',4'-4,5)imidazo(2,1-b)thiazol-Derivate der Formel

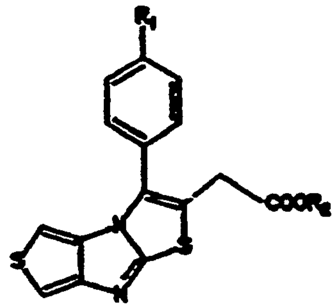

I,

in der
$R_1$ Wasserstoff, Halogen oder $CF_3$ und $R_2$ Wasserstoff oder $C_1$ - $C_4$ Alkyl bedeuten und, für den Fall, daß $R_2$ Wasserstoff bedeutet, ihre pharmazeutisch verwendbaren Salze.

2. Verbindungen der Formel I nach Anspruch 1 und ihre Salze, worin $R_1$ Chlor und $R_2$ Wasserstoff oder Methyl bedeuten.

3. 3-(4-Chlorphenyl)-thieno(3',4'-4,5)imidazo(2,1-b)thiazol-2-essigsäuremethylester. 3-(4-Chlorphenyl)-thieno(3',4'-4,5)imidazo(2,1-b)thiazol-2-essigsäure.

4. Verfahren zur Herstellung von Verbindungen der Formel I nach Anspruch 1 und ihren Salzen, dadurch gekennzeichnet, daß man

a) eine Verbindung der Formel

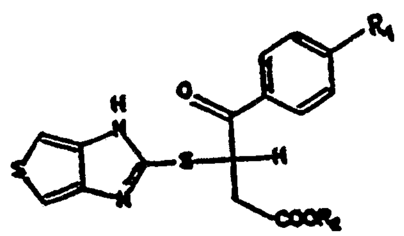

II,

in der
$R_1$ die obige Bedeutung hat und $R_2$ $C_1$ - $C_4$ Alkyl bedeutet, oder ein Säureadditionssalz davon, in Gegenwart von wasserentziehenden Reagenzien cyclisiert, worauf man

b) gegebenenfalls eine so erhaltene Verbindung der Formel I, in der $R_2$ $C_1$ - $C_4$ Alkyl bedeutet, zu Verbindungen der Formel I, in der $R_2$ Wasserstoff bedeutet, alkalisch verseift und

c) erwünschtenfalls die in Verfahrensschritt b) erhaltene freie Säure der Formel I mit anorganischen oder organischen Basen in ein pharmazeutisch verträgliches Salz überführt.

5. Pharmazeutische Präparate, enthaltend eine Verbindung der Formel I oder ein Salz davon in Kombination mit üblichen galenischen Hilfs- und/oder Trägerstoffen oder Verdünngungsmitteln.

6. Pharmazeutische Präparate, enthaltend eine Verbindung der Formel I oder ein Salz davon in Kombination mit anderen therapeutisch wertvollen Wirkstoffen sowie üblichen galenischen Hilfs-und/oder Trägerstoffen oder Verdünnungsmitteln.

7. Verbindung der Formel I oder Salze davon zur Verwendung als Wirkstoffe für Arnzeimittel zur Behandlung und Prophylaxe von Krankheiten, die durch ein defektes Immunsystem hervorgerufen werden.

Patentansprüche für folgende Vertragsstaaten: ES, GR

1. Verfahren zur Herstellung neuer Thieno-(3',4'-4,5)imidazo(2,1-b)thiazol-Derivate der Formel

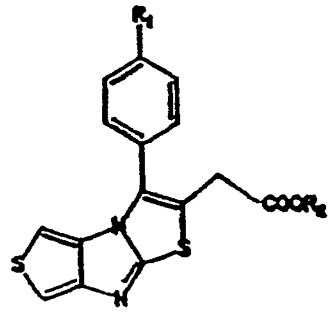

I,

in der
$R_1$ Wasserstoff, Halogen oder $CF_3$ und $R_2$ Wasserstoff oder $C_1$ - $C_4$ Alkyl bedeuten und, für den Fall, daß $R_2$ Wasserstoff bedeutet, ihrer pharmazeutisch verwendbaren Salze, dadurch gekennzeichnet, daß man

a) eine Verbindung der Formel

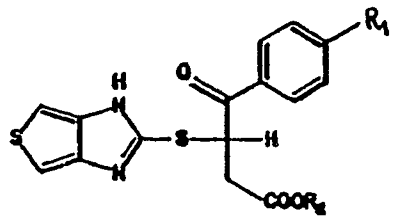

II,

in der
$R_1$ die obige Bedeutung hat und $R_2$ $C_1$ - $C_4$ Alkyl bedeutet, oder ein Säureadditionssalz davon, in Gegenwart von wasserentziehenden Reagenzien cyclisiert, worauf man

b) gegebenenfalls eine so erhaltene Verbindung der Formel I, in der $R_2$ $C_1$ - $C_4$ Alkyl bedeutet, zu Verbindungen der Formel I, in der $R_2$ Wasserstoff bedeutet, alkalisch verseift und

c) erwünschtenfalls die in Verfahrensschritt b) erhaltene freie Säure der Formel I mit anorganischen oder organischen basen in ein pharmazeutisch verträgliches Salz überführt.

2. Verfahren nach Anspruch 1, dadurch gekennzeichnet, daß man die Cyclisierung mit Polyphosphorsäure oder Phosphoroxychlorid als wasserentziehendem Reagens bei 60 - 110 °C für 5 Minuten bis 4 Stunden durchführt, wobei das wasserentziehende Reagens gleichzeitig als Lösungsmittel fungiert.

3. Verfahren nach einem der Ansprüche 1 oder 2, dadurch gekennzeichnet, daß die Cyclisierung mit Phosphoroxychlorid bei Rückflußtemperatur für 5 bis 30 Minuten erfolgt.

4. Verfahren nach einem der Ansprüche 1 bis 3, dadurch gekennzeichent, daß die Verseifung durch Kochen für 5 Minuten bis 1 Stunde mit äquivalenten Menge verdünnter Alkalyhydroxidlösungen unter Zusatz eines Lösungsvermittlers erfolgt.

5. Verfahren nach einem der Ansprüche 1 bis 4, dadurch gekennzeichent, daß die Verseifung in methanolischer Lösung mit verdünnter Natronlauge bei Rückflußtemperatur für 5 bis 25 Minuten erfolgt.

Tabelle 1

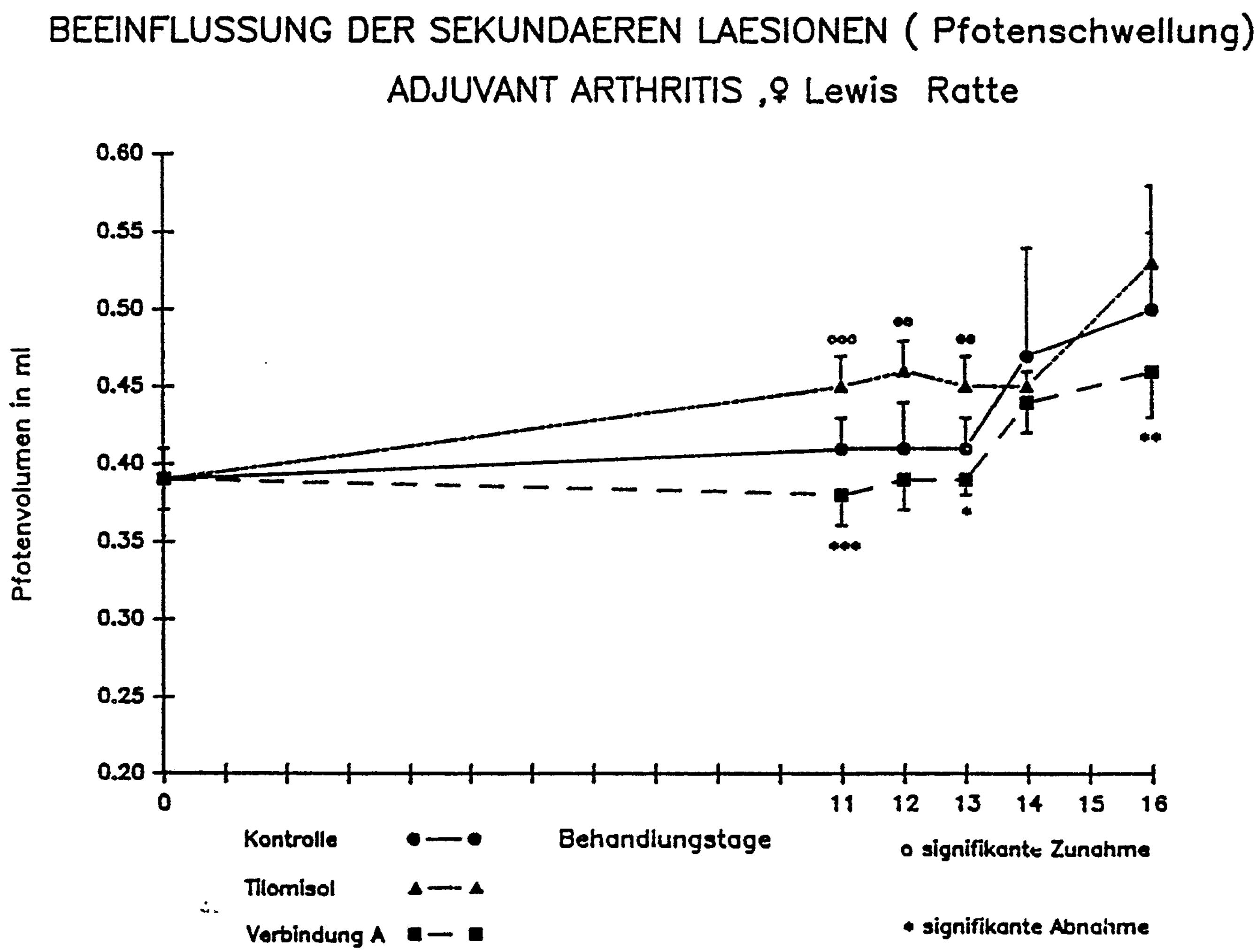
BEEINFLUSSUNG DER SEKUNDAEREN LAESIONEN ( Pfotenschwellung)
ADJUVANT ARTHRITIS ,♀ Lewis Ratte
Pfotenvolumen in ml
0.60
0.55
0.50
0.45
0.40
0.35
0.30
0.25
0.20
0
11
12
13
14
15
16
Behandlungstage
Kontrolle
Tilomisol
Verbindung A
o signifikante Zunahme
* signifikante Abnahme